(19) **Europäisches Patentamt / European Patent Office / Office européen des brevets**

(11) **EP 3 637 106 B1**

(12) # EUROPEAN PATENT SPECIFICATION

(45) Date of publication and mention
of the grant of the patent:
**22.03.2023   Bulletin 2023/12**

(21) Application number: **18813561.0**

(22) Date of filing: **07.06.2018**

(51) International Patent Classification (IPC):
**G01N 33/574** (2006.01)   **G01N 33/53** (2006.01)

(52) Cooperative Patent Classification (CPC):
**G01N 33/57423; C12Q 1/56;** G01N 2333/745

(86) International application number:
**PCT/JP2018/021901**

(87) International publication number:
**WO 2018/225830 (13.12.2018 Gazette 2018/50)**

(54) **LUNG CANCER DETECTION METHOD**

LUNGENKREBSNACHWEISVERFAHREN

PROCÉDÉ DE DÉTECTION DU CANCER DU POUMON

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **08.06.2017   JP 2017113289**

(43) Date of publication of application:
**15.04.2020   Bulletin 2020/16**

(73) Proprietors:
• **University of Miyazaki**
  **Miyazaki-shi**
  **Miyazaki 889-2192 (JP)**
• **Osaka University**
  **Suita-shi, Osaka 565-0871 (JP)**

(72) Inventors:
• **NAKAZATO, Masamitsu**
  **Miyazaki-shi**
  **Miyazaki 889-1692 (JP)**
• **MATSUMOTO, Nobuhiro**
  **Miyazaki-shi**
  **Miyazaki 889-1692 (JP)**
• **TSUBOUCHI, Hironobu**
  **Miyazaki-shi**
  **Miyazaki 889-1692 (JP)**
• **ARIMURA, Yasuji**
  **Miyazaki-shi**
  **Miyazaki 889-1692 (JP)**
• **YANAGI, Shigehisa**
  **Miyazaki-shi**
  **Miyazaki 889-1692 (JP)**
• **TAKAO, Toshifumi**
  **Suita-shi**
  **Osaka 565-0871 (JP)**
• **OKUMURA, Nobuaki**
  **Suita-shi**
  **Osaka 565-0871 (JP)**

(74) Representative: **Cabinet Beau de Loménie**
**158, rue de l'Université**
**75340 Paris Cedex 07 (FR)**

(56) References cited:
JP-A- 2015 105 951      JP-A- 2016 505 137
US-A1- 2010 008 935     US-A1- 2015 147 765

• DI MATTEI ET AL: "Occurrence of bradykinin in human pulmonary carcinoma", BIOCHEMICAL PHARMACOLOGY, ELSEVIER, US, vol. 16, no. 5, 1 May 1967 (1967-05-01), pages 909-911, XP025492566, ISSN: 0006-2952, DOI: 10.1016/0006-2952(67)90064-0 [retrieved on 1967-05-01]
• TRIFILIEFF A ET AL: "BRADYKININ BINDING SITES IN HEALTHY AND CARCINOMATOUS HUMAN LUNG", BRITISH JOURNAL OF PHARMACOLOGY, WILEY-BLACKWELL, UK, vol. 111, no. 4, 1 January 1994 (1994-01-01), pages 1228-1232, XP008032262, ISSN: 0007-1188

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DA COSTA PATRÍCIA L N ET AL: "The role of kinin receptors in cancer and therapeutic opportunities", CANCER LETTERS, NEW YORK, NY, US, vol. 345, no. 1, 11 December 2013 (2013-12-11), pages 27-38, XP028606192, ISSN: 0304-3835, DOI: 10.1016/J.CANLET.2013.12.009
- D. CHAN ET AL: "Bradykinin antagonist dimer, CU201, inhibits the growth of human lung cancer cell lines by a "biased agonist" mechanism", PROCEEDINGS OF THE NATIONAL ACADEMY OF SCIENCES, vol. 99, no. 7, 2 April 2002 (2002-04-02), pages 4608-4613, XP055564526, ISSN: 0027-8424, DOI: 10.1073/pnas.072077299
- CLAUDIA LANDI ET AL: "Proteome analysis of bronchoalveolar lavage in pulmonary langerhans cell histiocytosis", JOURNAL OF CLINICAL BIOINFORMATICS, BIOMED CENTRAL LTD, LONDON, UK, vol. 1, no. 1, 10 November 2011 (2011-11-10), page 31, XP021112615, ISSN: 2043-9113, DOI: 10.1186/2043-9113-1-31
- KITABATA, HIROKI: "Angiotensin-1-converting enzyme (ACE)", Japanese journal of clinical medicine, vol. 62, no. 11, 28 November 2004 (2004-11-28), pages 499-501, XP009518196, ISSN: 0047-1852
- MUNOZ, MIGUEL: "The Substance P/neurokinin-1 receptor system in lung cancer: Focus on the antitumor action of neurokinin-1 receptor antagonist", Peptides, vol. 38, no. 2, December 2012 (2012-12), pages 318-325, XP055219145,
- CHAN D, et al.: "Bradykinin antagonist dimer , CU 201, inhibits the growth of human lung cancer cell lines by a ''biased agonist'' mechanism", Proc Natl Acad Sci USA, vol. 99, no. 7, 2 April 2002 (2002-04-02), pages 4608-4613, XP055564526,
- KOSHIRO, MASATAKA: "Movements of Fibrinopeptide A (FPA), Fibrinopeptide Bbeta15-42 (PPBbeta), Factor XII, and Prekallikrein in blood with respect to malignant tumor", Medical Journal of Kagoshima University, vol. 40, no. 1, 1988, pages 17-24, XP009518207, ISSN: 0368-5063

Remarks:

The complete document including Reference Table(s) and the Sequence Listing(s) can be downloaded from the EPO website

**Description**

TECHNICAL FIELD

The present invention relates to a method for detection of lung cancer.

BACKGROUND ART

Lung cancer accounts for the largest number of yearly cancer deaths in the world. It is estimated that in 2012, 1.6 million people died of lung cancer worldwide. In Japan, 74,000 people each year die of lung cancer, which is the leading disease causing the death of cancer patients. Lung cancer is classified into small cell lung cancer and non-small cell lung cancer; the former accounts for 15% of all lung cancer patients and the latter accounts for the remaining 85%. Furthermore, non-small cell lung cancer can be pathologically classified into several types of cancer, such as adenocarcinoma, squamous cell carcinoma, large cell carcinoma, adenosquamous carcinoma, pleomorphic cancer, and sarcoma-like cancers such as carcinosarcoma.

The survival rate of patients with lung cancer decreases with the progression of clinical stage. For example, patients with non-small cell lung cancer at clinical stages IA, IB, IIA, IIB, and IIIA, on whom surgery can be performed, have a five-year survival rate of 82.0%, 66.1%, 54.5%, 46.1%, and 42.8%, respectively (Non-Patent Document 1), whereas patients at clinical stages IIIB and IV, to whom surgery is not applied, have a median survival time of 22.4 months (Non-Patent Document 2) and 10.3 months (Non-Patent Document 3), respectively.

As existing tumor markers for lung cancers, for example, serum Carcino embryonic antigen (CEA) for lung adenocarcinoma, a cytokeratin 19 fragment (CYFRA 21-1) for squamous cell lung cancer, and Neuron-specific enolase (NSE) for small cell lung cancer are employed for clinical diagnosis. However, the rate at which in cases of lung adenocarcinoma, CEA is positive for lung adenocarcinoma is only from 36.6 to 56.5% (Non-Patent Documents 4 and 5); especially cases of early lung adenocarcinoma at stage I give a decreased positive rate of 27% (Non-Patent Document 5). Thus, it is difficult that CEA is used to make a diagnosis of lung adenocarcinoma at disease stages allowing complete cure. The rate at which in cases of squamous cell lung cancer, CYFRA 21-1 is positive for squamous cell lung cancer is 57% (Non-Patent Document 6), and the rate at which in cases of small cell lung cancer, NSE is positive for small cell lung cancer is 45% (Non-Patent Document 7). Therefore, there is a need to develop a marker capable of detection of lung cancer with a higher sensitivity than that of the existing tumor markers.

Tumor markers that are being developed for clinical applications include, for example, serum CYBP (Patent Document 1) and UBE2L3 (Patent Document 2). These markers involve drawing blood from patients, and thus are invasive. Therefore, examinations using such markers are required to be less invasive.

PRIOR ART DOCUMENTS

PATENT DOCUMENTS

Patent Document 1: JP 2012-526976 W
Patent Document 2: JP 2014-115186 A

NON-PATENT DOCUMENTS

Non-Patent Document 1: Sawabata N, et al., J Thorac Oncol. 6: 1229-35, 2011.
Non-Patent Document 2: Atagi S, et al., Lancet Oncol. 13: 671-8, 2012.
Non-Patent Document 3: Scagliotti GV, et al., J Clin Oncol. 26: 3543-51, 2008.
Non-Patent Document 4: Molina R, et al., Am J Respir Crit Care Med. 193: 427-37, 2016
Non-Patent Document 5: Matsuoka K, et al., Eur J Cardiothorac Surg. 32: 435-9, 2007.
Non-Patent Document 6: Rastel D, et al., Eur J Cancer. 30A: 601-6, 1994.
Non-Patent Document 7: Stieber P, et al., Anticancer Res. 19 (4A): 2673-8, 1999.
Non-Patent Document 8 : Di Mattei et al., Biochemical Pharmacology, Elsevier, US, vol. 16, no. 5, 1 May 1967, pages 909-911, disclosing the use of bradykinin as a cleavage product of kininogen 1 for the diagnosis of lung cancer.
Non-Patent Document 9 : Trifilieff A et al., British Journal of Pharmacology, Wiley-Blackwell, UK, vol. 111, no. 4, 1 January 1994, pages 1228-1232, disclosing bradykinin binding sites in healthy and carcinomatous human lung.

Non-Patent Document 10 : Da Costa Patricia L N et al., Cancer Letter, New York, NY, US, vol. 345, nà. 1, 11 December 2013, pages 27-38, disclosing the role of kinin receptors in cancer and therapeutic opportunities.
Non-Patent Document 11 : D. Chan et al., Proceedings of the National Academy of Sciences, vol. 99, no. 7, 2 april 2003, pages 4608-4613, disclosing bradykinin antagonist dimer.
Non-Patent Document 12 : Claudia Landi et al., Journal of Clinical Bioinformatics, Biomed Central LTD, London, UK, vol. 1, no. 1, 10 November 2011, page 31, disclosing unusual peptide fragments of kininogen in bronchoalveolar lavge in pulmonary Langerhans cell histiocytosis.

## SUMMARY OF THE INVENTION

### PROBLEMS TO BE SOLVED BY THE INVENTION

The present invention aims to provide a method for detection of lung cancer.

### MEANS FOR SOLVING THE PROBLEMS

The present inventors have found an increase in the fragmentation of kininogen I and a decrease of a protein(s) having the normal structure derived therefrom in body fluids, and exposed portions due to nicks at abnormal sites or deletions at internal sites in a protein derived therefrom, which can be observed characteristically in patients with lung cancer. On the basis of these findings, the present inventors have conducted diligent investigations to establish a simple, minimally invasive or non-invasive method for detection of lung cancer, with the result that the present invention has been completed.

Accordingly, the present invention can be provided in the following items [1] to [6].

[Item 1]

A method for detection of lung cancer, comprising a step of detecting in vitro the presence or absence of abnormal cleavage of kininogen I in a subject-derived sample; wherein the step of detecting in vitro the presence or absence of abnormal cleavage of kininogen I comprises detecting the amount or presence of at least any one selected from the group consisting of:

(a) a protein fragment having ESNEELTESCET as a C-terminal amino acid sequence;
(b) a protein fragment having IYPTVNCQPLG as a C-terminal amino acid sequence;
(c) a protein fragment having TEHLASSSEDSTTPSA as a C-terminal amino acid sequence;
(d) a protein fragment having IYPTVNCQPLGMIS as a C-terminal amino acid sequence;
(e) a protein fragment having IYPTVNCQPL as a C-terminal amino acid sequence; and
(f) a protein fragment having IYPTVNCQP as a C-terminal amino acid sequence.

[Item 2]

The method for detection of lung cancer according to item 1, wherein the subject-derived sample is selected from urine or blood.

[Item 3]

The method for detection of lung cancer according to any one of items 1 and 2, comprising a step of using at least one method selected from the group consisting of a mass spectrometric method, an immunochemical method, and a chromatographic method.

[Item 4]

The method for detection of lung cancer according to any one of items 1 to 3, wherein the lung cancer is a non-small cell cancer selected from the group consisting of adenocarcinoma, squamous cell carcinoma, large cell carcinoma, adenosquamous carcinoma, pleomorphic cancer, and carcinosarcoma, or is small cell lung cancer.

[Item 5]

The method for detection of lung cancer according to item 1, wherein the presence or absence of abnormal cleavage in the kininogen I is determined by a fragmentation rate of the kininogen I, the fragmentation rate being given by:

$$\text{Protein fragmentation rate (Fn)} = Cn/In$$

where

"Cn" is the amount of each kininogen I protein fragment, and
"In" is the total amount of proteins derived from kininogen I protein.

[Item 6]

The method for detection of lung cancer according to any one of items 1 to 5, wherein the method is directed to any one or more selected from the group consisting of a detection for assisting the diagnosis of early lung cancer, a detection for the diagnosis of lung cancer, a detection of advanced lung cancer, a detection for predicting the presence or absence of recurred lung cancer, and a detection on the presence or absence of therapeutic effects against lung cancer.

EFFECT OF THE INVENTION

According to the present invention, lung cancer can be detected by a minimally invasive method using a body fluid such as urine and blood.

BRIEF DESCRIPTION OF THE DRAWINGS

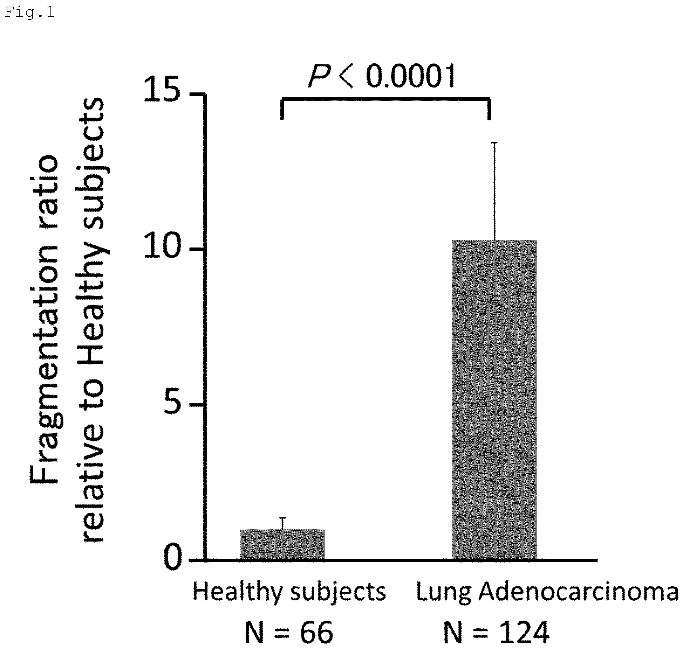

Fig. 1 represents a graph showing the ratio of amounts of a protein fragment having ESNEELTESCET as a C-terminal amino acid sequence after in vitro treatment of subject-derived samples (urine) from healthy subjects and from patients with lung adenocarcinoma.

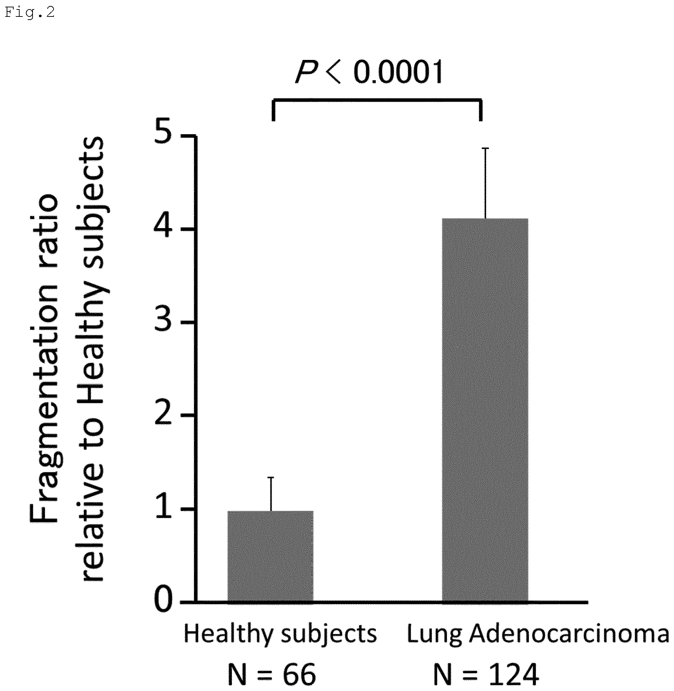

Fig. 2 represents a graph showing the ratio of amounts of a protein fragment having IYPTVNCQPLG as a C-terminal amino acid sequence after in vitro treatment of subject-derived samples (urine) from healthy subjects and from patients with lung adenocarcinoma.

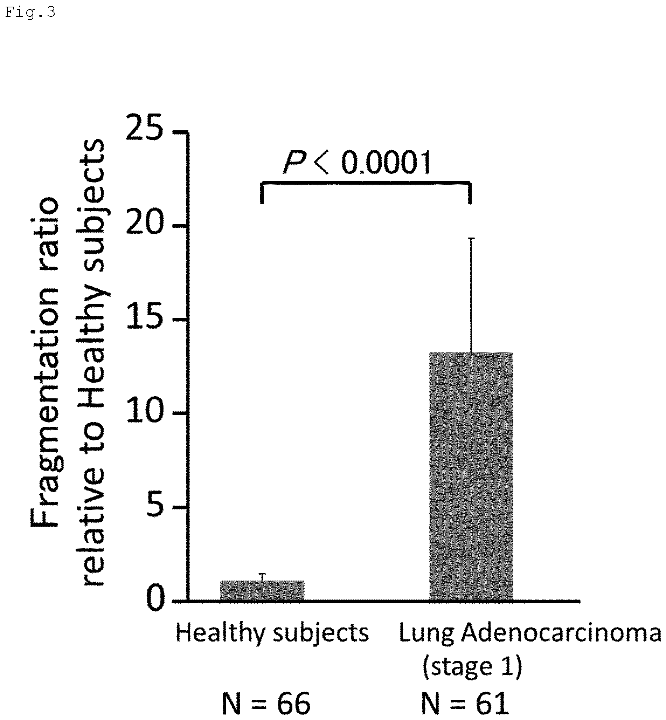

Fig. 3 represents a graph showing the ratio of amounts of a protein fragment having ESNEELTESCET as a C-terminal amino acid sequence after in vitro treatment of subject-derived samples (urine) from healthy subjects and from patients with early lung adenocarcinoma.

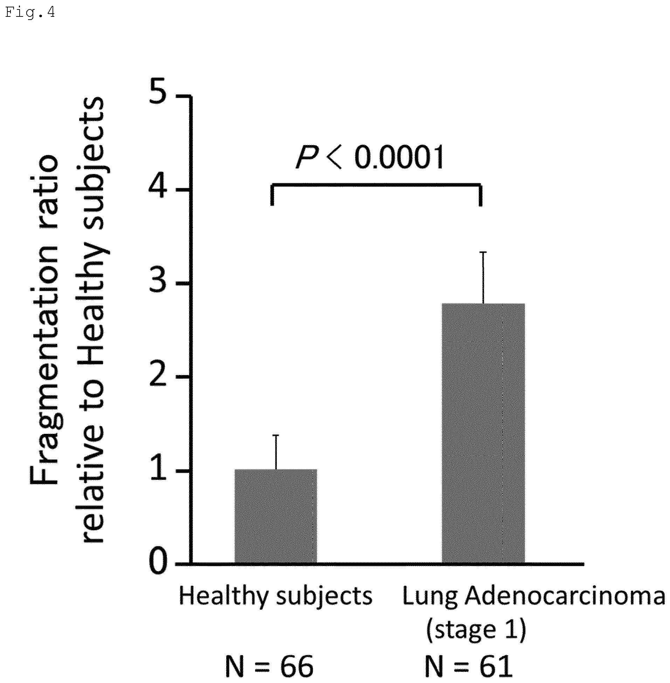

Fig. 4 represents a graph showing the ratio of amounts of a protein fragment having IYPTVNCQPLG as a C-terminal amino acid sequence after in vitro treatment of subject-derived samples (urine) from healthy subjects and from patients with early lung adenocarcinoma.
Fig. 5 represents a chest CT image in one case of lung adenocarcinoma in which a high level of a protein fragment having EYCGVPGDGDEEL as a C-terminal amino acid sequence was found by in vitro treatment of a subject-derived sample (urine). In the upper lobe S1 of the right lung, there are observed pleural invagination and bronchial transparency, and a primary lesion with a size of approximately 2 cm which is surrounded by a ground glass shadow.
Fig. 6 represents a PET-CT chest image in the same patient as in Fig. 5. Abnormal accumulation of PET signal is observed, consistent with the primary lesion found in the chest CT examination.
Fig. 7 represents images (of H-E staining and immunostaining with an antibody recognizing TTF-1) of a lung adenocarcinoma tissue sample collected by transbronchial biopsy from the same patient as in Fig. 5.
Fig. 8 represents (a) a graph showing changes over time in the fragmentation rate of kininogen I in one case of lung adenocarcinoma, and (b) a panel of chest CT images and a PET-CT image from the same case.
Fig. 9 represents a graph showing changes in the fragmentation rate before and after surgery in cases of early lung adenocarcinoma.
Fig. 10 represents chest CT images in one case of lung adenocarcinoma at stage IV who was positive for epidermal growth factor receptor (EGFR) gene mutation.

MODES FOR CARRYING OUT THE INVENTION

[Methods for detection of lung cancer]

A method for detection of lung cancer according to the present invention is based on a new discovery that was made by the present inventors. The present inventors have found that in the body of patients with lung cancer, kininogen I may be cleaved at a site or sites where the cleavage does not take place in healthy subjects, resulting in an increase of various types of deleted or nicked proteins derived therefrom.

In particular, the present inventors have found that in a specific protein, there is observed a phenomenon of abnormal cleavages leading to, for example, a deletion of a C-terminal or N-terminal portion, and nicks into the peptide bond at an abnormal position or a deletion of an amino acid(s) at the nicks associated therewith.

Abnormal cleavage of kininogen I may also be due to the presence of proteases specific for lung cancer. On the basis of these, the present invention makes it possible that patients with lung cancer are distinguished from healthy subjects and evaluated using in vitro, as an indicator, nicks or deletion of kininogen I due to such abnormal cleavage. It is also possible, on the other hand, that patients with lung cancer are distinguished from healthy subjects and evaluated using, as an indicator, an increased abnormal cleavage of kininogen I or a decreased amount of a normal protein (including the relative ratio of the amount thereof).

The present invention is directed to a method for detection of lung cancer, comprising a step of detecting in vitro the presence or absence of abnormal cleavage with respect to kininogen I in a subject-derived sample; wherein the step of detecting in vitro the presence or absence of abnormal cleavage of kininogen I comprises detecting the amount or presence of at least any one selected from the group consisting of:

(a) a protein fragment having ESNEELTESCET as a C-terminal amino acid sequence;
(b) a protein fragment having IYPTVNCQPLG as a C-terminal amino acid sequence;
(c) a protein fragment having TEHLASSSEDSTTPSA as a C-terminal amino acid sequence;
(d) a protein fragment having IYPTVNCQPLGMIS as a C-terminal amino acid sequence;
(e) a protein fragment having IYPTVNCQPL as a C-terminal amino acid sequence; and
(f) a protein fragment having IYPTVNCQP as a C-terminal amino acid sequence.

In the present specification, an "abnormal cleavage" of kininogen I leads to, without limitation to, a primary, secondary, or tertiary structure that is different from the normal structure of kininogen I. For examples, such an abnormal cleavage includes a cleavage that results in one or more nicks at peptide bonds in kininogen I, or a cleavage that results in a deletion of one or more than one amino acid residue at one or more sites in kininogen I.

An "abnormal cleavage" can generate, without limitation to, a protein having a deletion of an amino acid residue or residues on the C-terminal side of kininogen I, or a protein having a deletion of an amino acid residue or residues on the N-terminal side of kininogen I. Alternatively, an "abnormal cleavage" brings about nicks at the peptide bond between amino acid residues or a deletion of an amino acid(s) at any internal position in the protein.

In an embodiment of the "abnormal cleavage" of kininogen I, the peptide bond at an abnormal cleavage site is cleaved, whereby a new amino acid residue is exposed at the cleavage site, but the amino acid(s) that has/have been cleaved may not be away from the parent protein, for example, due to disulfide bonding. In this case, the deletion or fragmentation of the kininogen I may not be observed in a raw sample taken from the living body. The abnormal cleavage in the present invention includes this type of cleavage. Alternatively, although as mentioned, the parent protein maintains the binding structure, for example, by disulfide bonding and others, the peptide bond has been cleaved at an abnormal cleavage site, at which one or more than one amino acid residue may be further lost, thereby resulting in a deletion of an amino acid residue(s) within the protein at any internal position.

In regard to kininogen I, the intrinsic, normal post-translational processing is not encompassed by the abnormal cleavage described in the present invention.

In the present specification, a "subject" refers to a mammal of interest for detection of cancer, and is preferably, without limitation, a dog, a cat, a mouse, or a human.

In the present specification, lung cancer refers to a cancer that occurs in any tissue of the lung. The lung cancer can be a non-small cell cancer selected from the group consisting of adenocarcinoma, squamous cell carcinoma, large cell carcinoma, adenosquamous carcinoma, pleomorphic cancer, and carcinosarcoma, or can be small cell lung cancer. Among these, lung adenocarcinoma refers to a cancer that occurs in secretory gland tissues of the lung.

In the invention of the present application, the lung cancer may be at any stage of clinical stages IA, IB, IIA, IIB, and IIIA, and clinical stages IIIB and IV. When early lung cancer is referred to, early lung cancer is lung cancer at any of clinical stages IA, IB, IIA, and IIB.

The "detection of lung cancer" according to the present invention may be a detection for assisting the diagnosis of the presence or absence of the affection of lung cancer. Furthermore, the "detection of lung cancer" encompasses any of a detection of the presence or absence of or the degree of advanced lung cancer, a detection of the presence or absence of or the degree of recurred lung cancer, or a detection of the presence or absence of therapeutic effects or the degree of effects exerted by lung cancer surgery, anticancer drug treatment against lung cancer, and others. Here, the recurrence includes recurrence after surgery and after anticancer drug treatment. In addition, the presence or absence of therapeutic effects and the degree of treatment exerted are typically ones found, for example, in cancer reduction.

According to the method of the present invention, these detections can be easily performed. In other words, an "abnormal cleavage" of kininogen I underlying the present invention can be found in association with the affection of lung cancer. Furthermore, the ratio of abnormal cleavage of kininogen I may increase with the progression of lung cancer. Furthermore, abnormal cleavage of kininogen I may be found or increased with the recurrence of lung cancer. Furthermore, abnormal cleavage of kininogen I may decrease with effects exerted by lung cancer surgery and anticancer drug treatment.

In the present specification, a "subject-derived sample" refers to a body fluid derived from a subject, and represents, besides a body fluid itself, a concentration or dilution thereof, or other fluids treated as appropriate. In the present invention, the body fluid refers to, but is not limited to, for example, urine, blood (whole blood, plasma, and serum), sputum, sweat, cerebrospinal fluid, digestive juice, and ascites. Preferably, the body fluid is urine or blood, and particularly preferably urine. In the case of urine, the urine can be any of early morning midstream urine, pooled urine, and spot urine. The volume of a body fluid, such as urine and blood, to be collected is from 10 $\mu$l to 200 ml, preferably from 100 $\mu$l to 100 ml, further preferably from 1 ml to 100 ml.

A treatment of a body fluid refers more specifically to pretreatments such as concentration, dilution, fractionation, and desalting, and addition of preservatives such as glycerin, stabilizers such as protease inhibitors, antiseptics, and others. Also included are refrigerating or freezing a body fluid, followed by returning the body fluid to room temperature, and subjecting a body fluid to an appropriate treatment either before or after the body fluid is refrigerated or frozen. Furthermore, for example, in cases where the body fluid is blood, an anticoagulant treatment can be performed as an appropriate treatment. These treatments can be combined.

When the body fluid is urine, a sample of urine is preferably subjected to procedures which comprise concentrating the sample as a pretreatment prior to measurement. Methods for the concentration are not particularly limited, and include, for example, a method using an ultrafiltration membrane having a given molecular weight cutoff, freeze concentration, concentration under reduced pressure or in vacuum, and heating. The value of molecular weight cutoff is not limited, and can employ any value such as 3 kD, 10 kD, 30 kD, and 50 kD.

For example, when the body fluid is urine, the concentration of the total amount of proteins in raw urine samples is in the order of from 0.001g/dL to 0.6g/dL, and thus concentrating the samples is a useful pretreatment. Raw urine samples can be concentrated 200-250 times and used for analysis. The concentrated samples can be directly subjected to measurement, although they can also be diluted to adjust the total amount of proteins prior to measurement. For example, in measurement procedures such as Western blotting, the concentrated samples can be adjusted to have a total protein amount in the order of from 0.1 $\mu$g to 10 $\mu$g/$\mu$l prior to measurement.

For the concentration of samples, Vivaspin® (manufactured by Sartorius Japan K.K.), Amicon Ultra (manufactured by Merck Millipore), and the like can be used according to the manufacturer's instructions.

The dilution can be performed using distilled water or a buffer solution, and can also be used to prepare concentrated urine samples.

The deletion of kininogen I may be a deletion of an amino acid residue(s) in a C-terminal portion, in an N-terminal portion, or at an internal position of the full-length protein from which kininogen I is derived, upon comparison of their primary structures. Here, by the deletion product having a deletion in a C-terminal portion, in an N-terminal portion, or at an internal position is meant that the deletion product has a shorter C-terminal portion or N-terminal portion than the unit protein that usually provides normal functioning of the protein, or has lost an amino acid residue(s) to be located at the internal position.

In the present specification, when the sequence of a protein fragment is specified, each of the notation symbols used to describe the sequence means a usual letter used as a one-letter code of an amino acid residue.

Specifically,

A    Ala Alanine
C    Cys Cysteine
D    Asp Aspartic acid
E    Glu Glutamic acid
F    Phe Phenylalanine
G    Gly Glycine
H    His Histidine
I    Ile Isoleucine
K    Lys Lysine
L    Leu Leucine
M    Met Methionine
N    Asn Asparagine
P    Pro Proline
Q    Gln Glutamine
R    Arg Arginine

S     Ser Serine
T     Thr Threonine
V     Val Valine
W    Trp Tryptophan
Y     Tyr Tyrosine

In the present invention, the step of detecting in vitro the presence or absence of abnormal cleavage with respect to kininogen I in a subject-derived sample is limited to the following:
detecting the amount or presence of a protein fragment selected from the group consisting of:

(a) a protein fragment having ESNEELTESCET as a C-terminal amino acid sequence;
(b) a protein fragment having IYPTVNCQPLG as a C-terminal amino acid sequence;
(c) a protein fragment having TEHLASSSEDSTTPSA as a C-terminal amino acid sequence;
(d) a protein fragment having IYPTVNCQPLGMIS as a C-terminal amino acid sequence;
(e) a protein fragment having IYPTVNCQPL as a C-terminal amino acid sequence; and
(f) a protein fragment having IYPTVNCQP as a C-terminal amino acid sequence.

Also described but not claimed is an embodiment of the method for detection of lung cancer which comprises detecting a decrease in the amount of one or more than one protein having the normal structure from kininogen I, in a subject-derived sample. Specifically, for example, the amount of kininogen I having the normal structure is determined by the presence or absence of the amino acid sequence of a C-terminal portion and/or of an N-terminal portion of the kininogen I, whereby a detection can be made as to whether or not the amount of a protein having the normal structure is decreased relatively, compared with that in the case of healthy subjects.

Also described but not claimed is the method for detection of lung cancer comprising detecting a protein fragment from kininogen I in vitro using a subject-derived sample. The detecting of the protein fragment can be to detect a protein fragment that exists in a fragmented state in the subject-derived sample and/or a protein fragment that is generated, for example, after the subject-derived sample is subjected to reduction treatment or the like.

In the present specification, when a C-terminal sequence of a protein fragment from kininogen I is described, the fragment is expressed, for example, as a "protein fragment having ESNEELTESCET as a C-terminal amino acid sequence," a "protein fragment having IYPTVNCQPLG as a C-terminal amino acid sequence," and a "protein fragment having TEHLASSSEDSTTPSA as a C-terminal amino acid sequence." For example, a "protein fragment with ESNEELTESCET as a C-terminal amino acid sequence" describes that the protein fragment has a deletion of (is devoid of) the amino acid residues that are originally located in the full-length protein on the C-terminal side from the residue T indicated at the right end, and means that the protein fragment may or may not have the amino acid residues that are located on the N-terminal side from the first residue E.

The protein fragment from kininogen I detected in the method of the invention is at least one or more selected from the group consisting of:

(a) a protein fragment having ESNEELTESCET as a C-terminal amino acid sequence (also referred to herein as P1) ;
(b) a protein fragment having IYPTVNCQPLG as a C-terminal amino acid sequence (also referred to herein as P2) ;
(c) a protein fragment having TEHLASSSEDSTTPSA as a C-terminal amino acid sequence;
(d) a protein fragment having IYPTVNCQPLGMIS as a C-terminal amino acid sequence;
(e) a protein fragment having IYPTVNCQPL as a C-terminal amino acid sequence; and
(f) a protein fragment having IYPTVNCQP as a C-terminal amino acid sequence.

Furthermore, the method allows detection of early lung cancer, such as early lung adenocarcinoma, by determining in vitro at least one or more selected from the group consisting of these protein fragments from kininogen I.

In the present invention, the number of amino acid residues in a protein fragment from kininogen I is not particularly limited, and is preferably at least 10 residues or more, and more preferably at least 50 residues or more. The upper limit of the size of the protein fragment is not particularly limited, and is less than the size of the full-length protein from which each protein fragment is derived. For example, but without limitation, the protein fragment is one having a structure in which the amino acid residues on the C-terminal side are deleted relative to the full-length protein from which each protein fragment is derived. The protein fragment is not particularly limited, as long as the protein fragment has a size less than the full length, and in some cases, can be present as a short fragment having such a size as 200 residues or less, preferably 100 residues or less.

Furthermore, the method also makes it possible to detect the presence of or an increase in the relative amount of a protein having a deletion of amino acid residues on the N-terminal side of kininogen I, in a subject-derived sample; or to detect a released protein fragment on the C-terminal side having a deletion of the N-terminal-side portion, in order to detect the presence of or an increase in the relative amount of a cleavage or deletion at any internal site in the amino acid sequence, with respect to kininogen I in a subject-derived sample. The detecting of such a fragment allows indirect detection of the protein fragments (a) to (f), or detection of the presence of a protein fragment in which the N-terminus portion is deleted within the body.

The molecular weight of a protein fragment from kininogen I of the present invention is not limited, as long as the protein fragment has a molecular weight less than the full-length protein. However, depending on methods in which a sample such as urine is used to detect lung cancer, the protein fragment has a molecular weight of, for example, 3 kDa or more and less than that of the full length protein, preferably 10 kDa or more and less than that of the full length protein.

Furthermore, these protein fragments from kininogen I each includes ones that have undergone post-translational modifications such as glycosylation and phosphorylation, as long as each of them has the above-mentioned sequences.

Explanation is now made with illustrating the sequences of protein fragments from kininogen I as a specific sequence contained in a given database (SEQ ID NO:1 or 2 in the sequence listing). As will be apparent to those skilled in the art, these sequences, and the positions and number of amino acid residues therein may vary from individual to individual, and a different sequence may be deposited in a different database.

Kininogen I is a protein that plays an important role in the kallikrein-kinin system. This metabolic system is a series of in vivo reactions that generates kinins having various physiological activities.

The kallikrein-kinin system is involved in the regulation of biological functions in relation to various enzymatic reaction systems within the body, such as the renin-angiotensin-aldosterone system and the blood coagulation cascade. Kinins such as bradykinin, which is a product from kininogen I, exhibit a variety of physiological activities such as decreased blood pressure associated with peripheral vasodilatation, increased vascular permeability, contraction or relaxation of smooth muscle, pain generation, and leukocyte migration. Kininogen I is a protein that is a precursor of this kinin and is an endogenous blood-coagulation factor. Kininogen I is present in very high concentrations in the female genitals, and it is supposed that by releasing bradykinin, kininogen I has an important role in pregnancy and delivery through the kallikrein-kinin system. Kininogen I is also suggested to be associated with recurrent miscarriage.

Kininogen I has, as a precursor sequence, the sequence shown, for example, in UniProtKB-P01042 (SEQ ID NO:1 in the sequence listing) or UniProtKB-P01042-2 (SEQ ID NO:2 in the sequence listing).

Kininogen I has several typical isoforms (high and low molecular weight forms). The high molecular weight form (SEQ ID NO:1 in the sequence listing) is synthesized as a precursor consisting of 644 amino acids with a signal peptide from amino acid numbers 1 to 18. The mature form (amino acid positions 19 to 644) is further processed into a heavy chain (amino acid positions 19 to 380) and a light chain (amino acid positions 390 to 644), thereby to release bradykinin (amino acid positions 381 to 389). In addition, there can be cleavage into T-kinin (amino acid positions 376 to 389), lysyl-bradykinin (amino acid positions 380 to 389), or the like, and into a low molecular weight growth-promoting factor (GPF) (amino acid positions 431 to 434). The low molecular weight form has been reported to have mainly three sequences. The sequence shown in SEQ ID NO:2 in the sequence listing is the same in the sequences of the heavy chain and the bradykinin segment as those in the high molecular weight form and is different in the sequence of the light-chain. The sequence shown in SEQ ID NO:3 in the sequence listing has a deletion of amino acids from positions 189 to 224 of the sequence shown in SEQ ID NO:2 in the sequence listing. The sequence shown in SEQ ID NO:4 in the sequence listing has a substitution of 17 amino acids for amino acids from positions 1 to 153 of the sequence shown in SEQ ID NO:2 in the sequence listing and additionally a substitution of Thr for Met at position 177. None of these low molecular weight forms is known to be involved in the blood coagulation cascade. In the present specification, an example of the amino acid sequence of the high molecular weight form is disclosed in SEQ ID NO: 1 in the sequence listing, and examples of the amino acid sequences of the low molecular weight forms are disclosed in SEQ ID NOS: 2 to 4 in the sequence listing.

A "protein having the normal structure from kininogen I" and "kininogen I having the normal structure" as used herein release a mature form (amino acids from positions 19 to 644 in SEQ ID NO:1 in the sequence listing, amino acids from positions 19 to 427 in SEQ ID NO:2 in the sequence listing, amino acids from positions 19 to 391 in SEQ ID NO:3 in the sequence listing, amino acids from positions 1 to 291 in SEQ ID NO:4 in the sequence listing), a heavy chain (amino acids from amino acid positions 19 to 380 with reference to SEQ ID NO:1 or 2 in the sequence listing), a light chain (amino acids from amino acid positions 390 to 644 in SEQ ID NO:1 in the sequence listing or amino acids from amino acid positions 390 to 427 in SEQ ID NO:2 in the sequence listing, or corresponding amino acids in SEQ ID NO:3 to 4 in the sequence listing), and bradykinin (amino acids from amino acid positions 381 to 389 in SEQ ID NO:1 or 2 in the sequence listing, or corresponding amino acids in SEQ ID NO:3 to 4 in the sequence listing). In addition, there can be cleavage into T-kinin (amino acids from amino acid positions 376 to 389 in SEQ ID NO:1 or 2 in the sequence listing), lysyl-bradykinin (amino acids from amino acid positions 380 to 389 in SEQ ID NO:1 or 2 in the sequence listing), or the like and into a low molecular weight growth-promoting factor (GPF) (amino acids from amino acid positions 431 to 434 in SEQ ID NO:1 in the sequence listing). These cleavage products each have the normal structure.

As used herein, a "cleavage at an abnormal position" refers to a cleavage into a signal peptide and a mature form, and a cleavage into a chain that is not a chain selected from the group consisting of a heavy chain, a light chain, bradykinin, T-kinin, lysyl-bradykinin, and a low molecular weight growth-promoting factor (GPF). These may be because kininogen I may go through cleavage by enzymes having increased activities in lung cancer such as matrix metalloproteases, whereby cleavage occurs at positions at which cleavage does not occur by nature, resulting in a secondary increase in fragmentation and deletion of amino acids.

The protein fragment from kininogen I that can be detected as an example of the abnormal cleavage described herein, when explained with reference to the sequence shown in SEQ ID NO:1 or 2 in the sequence listing, may be a protein fragment having a C-terminal portion of amino acid residues identical to those from positions 331 to 342 and a deletion of the C-terminal portion extending from position 343. The protein fragment from kininogen I that can be detected as another example of the abnormal cleavage described herein, when explained with reference to the sequence shown in SEQ ID NO:1 or 2 in the sequence listing, may be a protein fragment having a C-terminal portion of amino acid residues identical to those from positions 364 to 374 and a deletion of the C-terminal portion extending from position 375. In another embodiment, the protein fragment from kininogen I that can be detected as an example of the abnormal cleavage described herein, when explained with reference to the sequence shown in SEQ ID NO:1 in the sequence listing, may be a protein fragment having a C-terminal portion of amino acid residues identical to those from positions 521 to 536 and a deletion of the C-terminal portion extending from position 537. In yet another embodiment, the protein fragment from kininogen I that can be detected as another example of the abnormal cleavage described herein, when explained with reference to the sequence shown in SEQ ID NO:1 or 2 in the sequence listing, may be a protein fragment having a C-terminal portion of amino acid residues identical to those from positions 364 to 377 and a deletion of the C-terminal portion extending from position 378. In yet another embodiment, the protein fragment from kininogen I that can be detected as another example of the abnormal cleavage described herein, when explained with reference to the sequence shown in SEQ ID NO:1 or 2 in the sequence listing, may be a protein fragment having a C-terminal portion of amino acid residues identical to those from positions 364 to 373 and a deletion of the C-terminal portion extending from position 374. In still another embodiment, the protein fragment from kininogen I that can be detected as another example of the abnormal cleavage described herein, when explained with reference to the sequence shown in SEQ ID NO:1 or 2 in the sequence listing, may be a protein fragment having a C-terminal portion of amino acid residues identical to those from positions 364 to 372 and a deletion of the C-terminal portion extending from position 373.

In the method for detecting an abnormal cleavage according to the present invention, the amount of or the presence of kininogen I having the normal structure and of a protein fragment from kininogen I can be detected in terms of the mass, molecular weight, concentration, or others of the kininogen I and protein fragment therefrom contained in a body fluid which is a subject-derived sample. Furthermore, the amount of or the presence of the kininogen I and protein fragment therefrom can also be described in terms of fluorescence intensity, absorbance, intensity in MS/MS spectra, and the like.

A detection method used in the present invention is not particularly limited, as long as the method is a method for detecting an abnormal cleavage or a method for detecting a protein having the normal structure contained in a subject-derived sample. Such a method can include, for example, besides methods in which the detecting is carried out by mass spectrometry, methods in which the detecting is carried out by immunochemical procedures using antibodies (radioimmunoassay, enzyme immunoassay, Western blotting, and others).

A detection method using mass spectrometry is not particularly limited, as long as the method is a method for detecting kininogen I having the normal structure or a protein fragment therefrom resulting from an abnormal cleavage, in a subject-derived sample. Specifically, for example, included is a method in which a sample is collected and subjected to pretreatment and/or reduction treatment, followed by enzymatic digestion, followed by labeling with an isotope or the like, and then determination of only the C-terminus-derived peptide by MS/MS. More specifically, but without limitation, when the subject-derived sample is urine, a sample of urine is collected, concentrated as a pretreatment to obtain a concentrated sample of the urine sample, which is then subjected to reductive alkylation, followed by tryptic digestion in the presence of $H_2^{18}O$, making it possible that peptides resulting from the tryptic digestion are labeled with the stable isotope. Then, the labeled products are desalted and purified by ion exchange chromatography before only the trypsin-digested peptide derived from the C-terminus is determined by Nano-LC-MALDI-MS/MS. An analysis of the information on this peptide allows differential detection of a protein having the normal structure or a protein fragment from kininogen I resulting from an abnormal cleavage. Alternatively, for example, a method can be employed in which proteins in the concentrated sample is subjected to enzymatic digestion with Lys-C, and then the N-terminus of all digested peptides is blocked with such a reagent as phenyl isocyanate or TMPP reagent (N-succinimidyloxycarbonyl-methyl)tris(2,4,6-trimethoxyphenyl)phosphonium bromide) to obtain peptides in which the amino acid at the C-terminus is other than lysine, which are then subjected to MS/MS analysis using a mass spectrometer, thereby to determine a protein fragment from kininogen I.

In cases where the amount of a protein fragment from kininogen I is determined by MRM (Multiple Reaction Monitoring), use can be made of, for example, a high-performance liquid chromatography/triple quadrupole mass spectrometer (QTRAP(R) 5500 System (AB Sciex) and an LCMS-8030 (manufactured by Shimadzu Corporation).

For methods using radioimmunoassay and enzyme immunoassay, antibodies are desirably used that can specifically recognize an exposed amino acid resulting from an abnormal cleavage, a C-terminal portion of a protein fragment from kininogen I, or a specific portion having a deletion of an amino acid(s), and distinguish the abnormal-cleavage product from a protein having the corresponding normal structure.

The amount of kininogen I having the normal structure or of a protein fragment from kininogen I in a subject-derived sample can be compared with the abundance of all kininogen I-derived proteins coexisting in the sample, and expressed as a relative ratio to the abundance. Alternatively, the abundance of a protein fragment in a subject-derived sample can be compared with the total amount of proteins in the sample, and can be expressed as a relative ratio of the amount of the fragment to the total amount of proteins.

In cases where any of these measurement procedures is used, the presence or absence of abnormal cleavage in kininogen I can be determined by calculating the fragmentation rate of kininogen I in the subject's sample and on the basis of whether or not its value exceeds a given threshold. Accordingly, if the rate of protein fragmentation of kininogen I exceeds a given threshold, then the subject from which a sample having the protein fragment detected therein was taken can be determined to be affected by lung adenocarcinoma.

More specifically, but without limitation, for example, the fragmentation rate ($F_n$) of kininogen I is determined by the formula described below, and a patient's sample having a fragmentation rate that is 1.5 times or more higher than the mean value of a healthy subjects group can be considered to be positive for lung adenocarcinoma.

$$[Formula\ 1]\ Protein\ fragmentation\ rate\ (F_n)\ =\ C_n/I_n$$

where

$C_n$: the amount of each protein fragment from kininogen I, and
$I_n$: the total amount of proteins derived from the kininogen I protein

The amount of each fragment from kininogen I represented as $C_n$ refers to the amount of any one of the protein fragments (a) to (f).

The amount of the parent protein represented as $I_n$ refers to the total amount of any one of the protein fragments (a) to (f) and the full-length protein having the normal structure. The amount represented as $C_n$ or $I_n$ can be detected by, for example, determining the amount of a trypsin-digested peptide. The $I_n$ can be indicated by the amount of a trypsin-digested peptide that is contained in common in both trypsin-digested products of a protein fragment from kininogen I and of kininogen I having the normal structure. The method for determination is carried out under conditions conforming to those described in Example 5.

The sequence included in common in digestion peptides is not limited, and can use the sequence described below:
YFIDFVAR (positions 317 to 324 in SEQ ID NO: 1 in the sequence listing)

If the amount of a protein having the normal structure in a subject-derived sample is significantly decreased when compared with that in a sample from a healthy subject (a healthy sample), then the subject examined is determined to be a patient with lung cancer. Here, a significant decrease means that for example, the relative ratio of the protein having the normal structure is not more than 0.9 times, preferably not more than 0.8 times, more preferably not more than 0.6 times, compared to a healthy subject's sample. Alternatively, when an antibody is used, a significant decrease means that the decrease in the amount that can be recognized, for example, from the intensity of the label is in the order of not more than 0.8 times, preferably not more than 0.6 times, compared to a healthy subject's sample.

If the amount of a protein fragment from kininogen I in a subject-derived sample is compared with that in a sample from a healthy subject (a healthy sample) and a significant increase of the protein fragment is detected, then the subject examined is determined to be a patient with lung cancer. Here, a significant increase means that for example, in terms of the MS/MS spectrum area, the intensity is 1.25 times or more, preferably 1.5 times or more, more preferably 2.0 times or more, compared to a healthy subject's sample. Alternatively, when an antibody is used, a significant increase means that the increase in the amount of the protein fragment that can be recognized, for example, from the intensity of the label is a value of more than 1, and is in the order of preferably 1.2 times or more, more preferably 1.5 times or more, compared to a healthy subject's sample.

In these cases, numerical values for comparison are intended to represent values after correction using an internal standard or the like.

The amount of kininogen I having the normal structure or of a protein fragment from kininogen I in a healthy subject-derived sample, and others can be determined according to the methods for preparation and measurement of subject-derived samples.

In a particularly preferred embodiment of the present invention, the step of detecting in vitro an abnormal cleavage comprises detecting a decrease in the amount of a protein having the normal structure derived from kininogen I which is present in the subject's sample, and/or the amount or presence of a protein fragment derived from kininogen I. In this embodiment, antibodies can be suitably used which recognize, for example, (a) a C-terminal portion of a protein fragment having ESNEELTESCET as the amino acid sequence of a C-terminal portion; (b) a C-terminal portion of a protein fragment having IYPTVNCQPLG as the amino acid sequence of a C-terminal portion; (c) a C-terminal portion of a protein fragment having TEHLASSSEDSTTPSA as a C-terminal amino acid sequence; (d) a C-terminal portion of a protein fragment having IYPTVNCQPLGMIS as a C-terminal amino acid sequence; (e) a C-terminal portion of a protein fragment having IYPTVNCQPL as a C-terminal amino acid sequence; and (f) a C-terminal portion of a protein fragment having IYPTVNCQP as a C-terminal amino acid sequence.

[Antibodies]

Also described but not claimed is an antibody composition for detection of lung cancer, comprising an antibody capable of determining a protein fragment from kininogen I in a subject-derived sample. An antibody that is used for determining a protein fragment from kininogen I is not limited, as long as the antibody is an antibody that can recognize the protein fragment from kininogen I, with preference being given to an antibody that can specifically recognize the C-terminal-side cleavage of the protein fragment from kininogen I and does not recognize other proteins such as the full-length protein. The antibody can be a polyclonal antibody or a monoclonal antibody, as long as the antibody can specifically recognize a protein fragment from kininogen I. The antibody may be immobilized on a solid phase.

EXAMPLES

[Example 1] Collection of body fluids

From among patients having an intrapulmonary nodular or mass shadow suspected of primary lung cancer on chest imaging examination, patients were selected who were diagnosed with lung adenocarcinoma by surgery, transbronchial biopsy, lymph node biopsy, or cytology. Samples of early morning midstream urine were collected from the selected patients with lung adenocarcinoma and from healthy subjects using sterile cups. The urine samples collected were stored at -80°C until analysis.

A comprehensive analysis was made of C-terminal protein fragments in samples of urine from 124 cases of lung adenocarcinoma cases and 66 healthy subjects. The clinical stages of the lung adenocarcinoma cases were stage IA for 46 cases, stage IB for 15 cases, stage IIA for 1 case, stage IIB for 1 case, stage IIIA for 8 cases, stage IIIb for 8 cases, and stage IV for 45 cases. The clinical stages were determined according to the criteria described in "General Rule for Clinical and Pathological Record of Lung Cancer, 7th Edition" edited by The Japan Lung Society (published by Kanehara & Co., Ltd.). The cases of lung adenocarcinoma at stages IA and IB were defined as those of early lung adenocarcinoma.

[Example 2] Detection by mass spectrometry

Samples of urine (~50 mL) were collected and markers were searched in a sequence of sample pretreatment, and obtaining and statistical analysis of analytical date. A total of two markers were identified as diagnostic markers for lung adenocarcinoma and early lung adenocarcinoma (Tables 1 and 2).

[Table 1]

| Protein name | Protein abbreviation | Sequence of protein fragment | Relative ratio to healthy subjects | Sensitivity (%) | Specificity (%) | ROC-AUC | p-value |
|---|---|---|---|---|---|---|---|
| KNG1 fragment group | | | | | | | |
| Kininogen 1 | KNG1 | ESNEEL TESCET | 10.331 | 42.74 | 87.88 | 0.744 | < 0.0001 |
| Kininogen 1 | KNG1 | IYPTVNCQPLG | 4.124 | 54.84 | 92.42 | 0.814 | < 0.0001 |

[Table 2]

| Protein name | Protein abbreviation | Sequence of protein fragment | Relative ratio to healthy subjects | Sensitivity (%) | Specificity (%) | ROC-AUC | p-value |
|---|---|---|---|---|---|---|---|
| KNG1 fragment group | | | | | | | |
| Kininogen 1 | KNG1 | ESNEEL TESCET | 13.209 | 29.51 | 87.88 | 0.676 | < 0.0001 |
| Kininogen 1 | KNG1 | IYPTVNCQPLG | 2.762 | 42.62 | 92.42 | 0.766 | < 0.0001 |

[Example 3] Evaluation methods for lung adenocarcinoma

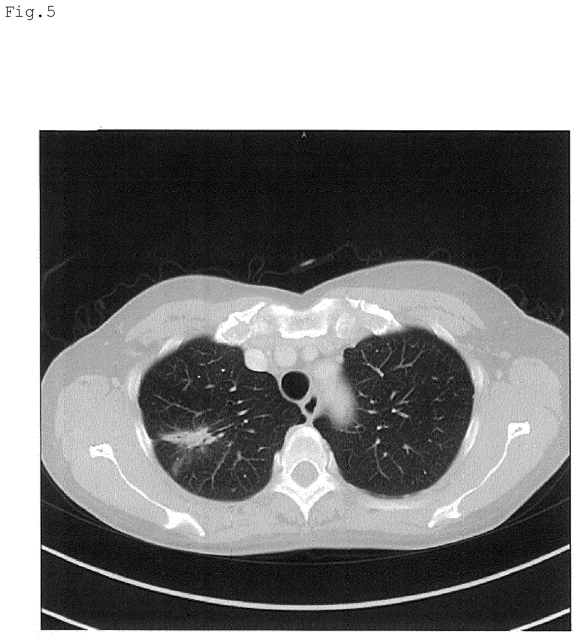

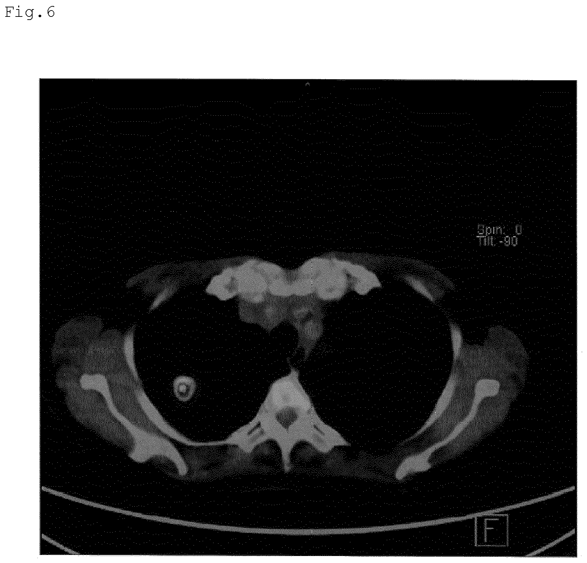

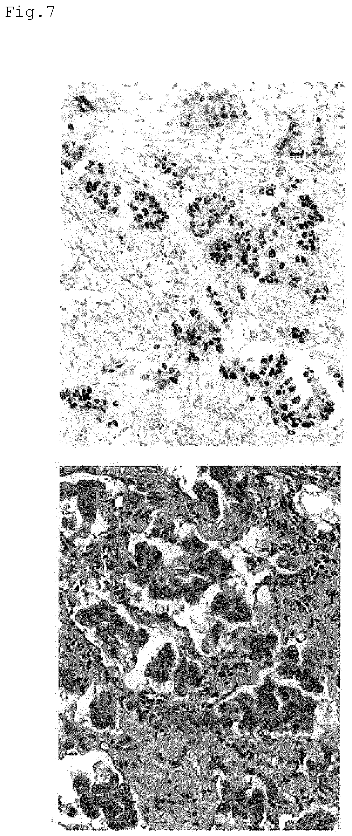

In patients having an intrapulmonary nodular or mass shadow suspected of primary lung cancer on chest imaging examination, lung adenocarcinoma was diagnosed by surgery, histological examination, such as lung biopsy, lymph node biopsy, or bone biopsy, and cytological examination of samples of sputum, pleural effusion, and others. For the determination of the clinical stage of lung adenocarcinoma, bone biopsy, fine-needle aspiration cytology of lymph nodes, or cytological examination of pleural effusions was employed besides accumulated intensity in PET-CT scans, and imaging examination by head MRI and chest CT scans. In one case of lung adenocarcinoma who exhibited in Example 2, a high level of a urine protein fragment in which the sequence of C-terminal amino acids derived from kininogen I was IYPTVNCQPLG, there was observed a 2-cm nodular shadow in the right upper lobe in a chest CT scan (Fig. 5), which had an abnormal accumulation of intensity observed in a PET-CT scan (Fig. 6). Lung adenocarcinoma tissues positive for thyroid transcription factor-1 (TTF-1) were detected from tissues obtained by transbronchial biopsy (Fig.7). In this case, the spectral area of the protein fragment in which the sequence of C-terminal amino acids derived from kininogen I was IYPTVNCQPLG was 11.46 times the mean value of the healthy subjects.

[Example 4] Comparison with CEA for usefulness in diagnosis of lung adenocarcinoma

Out of 124 cases diagnosed with lung adenocarcinoma (Example 1), 116 cases of lung adenocarcinomas in which the blood CEA values were determined were subjected to comparison for the usefulness in the diagnosis of lung adenocarcinoma between CEA and a urine protein fragment. For example, if a protein fragment in which the sequence of C-terminal amino acids derived from kininogen I was IYPTVNCQPLG exhibiting 1.5 times the mean value of the healthy subjects, in Example 2 was considered to be positive for lung adenocarcinoma, then 61 cases out of the 116 cases of lung adenocarcinoma were allowed to be assessed to be positive for lung adenocarcinoma. On the other hand, when the reference value of the blood CEA value was set to be 8.0 ng/ml, only 45 cases out of the 116 cases of lung adenocarcinoma gave a positive result.

[Example 5] Screening

Samples were prepared and subjected to an MRM method, thereby to screen lung adenocarcinoma patients. Samples of urine (~50 mL) were collected, followed by sample pretreatment and obtaining and analysis of analytical data. Specifically, sample pretreatment was performed to concentrate the urine samples 200-250 times using an Amicon Ultra-15 (having a molecular weight cut-off of 10 kDa) and an Amicon Ultra-4 (having a molecular weight cut-off of 10 kDa) (Merck Millipore), and remove low molecular weight molecules by washing, prior to obtaining concentrated samples. The concentrated samples were subjected to protein quantification, and all the samples were adjusted to have a protein concentration, or a total protein amount, of 10 mg/mL using a buffer solution, and used in a subsequent analysis step. Subsequently, the sample was subjected to reductive alkylation, followed by tryptic digestion in a buffer solution. After desalting and purification, trypsin-digested peptides derived from protein fragments were fractionated by ion-exchange chromatography (on an LC column of PolySULFOETHYL A™ (PolyLC Inc., USA) having an inner diameter of 4.6 mm and a length of 50 mm, at a flow rate of 0.4 mL/min, using as a solvent, a stepwise gradient (from 0 to 100%) of 20% acetonitrile/aqueous solution of 5 mM monopotassium phosphate and 0.5M NaCl (pH2.55) in 20% acetonitrile/aqueous phosphoric acid (pH2.55) for separating), thereby obtaining a mixture of trypsin-digested peptides containing the C-terminal peptide of the protein fragment. The samples thus obtained was each individually subjected to a high performance liquid chromatography/triple quadrupole mass spectrometer (a QTRAP(R) 5500 System (AB Sciex) or an Agilent 6470 LC/MS/MS System), whereby the separation and at the same time, the quantification of the peptides in the mixture were performed by the MRM method (Multiple Reaction Monitoring). The fragmentation rate ($F_n$) of each protein was determined by the formula described below, and a patient's sample having a fragmentation rate that was 1.5 times or more higher than the mean value of the healthy subjects group was considered to be positive for lung adenocarcinoma.

$$[\text{Formula 1}]\ \text{Protein fragmentation rate }(F_n) = C_n/I_n$$

where

C$_n$: the amount of each protein fragment (amount of peptides having the sequences of protein fragments (a) to (f)), and
I$_n$: the amount of the parent protein derived from each protein fragment

Here, the amount of the C-terminal fragment of each protein represented as $C_n$ refers to the amount of a protein fragment from kininogen I. The amount of the parent protein represented as $I_n$ refers to the amount of a trypsin-digested peptide that is contained in common in protein fragments or in the full-length protein. (A trypsin-digested peptide included in common is YFIDFVAR).

Samples of urine from 124 cases of lung adenocarcinoma and 66 healthy subjects were subjected to an MRM method, thereby to determine the fragmentation rate of a protein fragment in which the sequence of C-terminal amino acids derived from kininogen I was ESNEELTESCET. When a patient's sample having a fragmentation rate that was 1.25 times or more higher than the mean value of the healthy subjects group was considered to be positive for lung adenocarcinoma, 8 cases out of the healthy subjects and 61 cases out of the lung adenocarcinoma cases were positive for lung adenocarcinoma. When as a reference, the fragmentation rate was set to be 1.5 times the mean value of the healthy subjects group, 8 cases out of the healthy subjects and 53 cases out of the lung adenocarcinoma cases were positive for lung adenocarcinoma, and the sensitivity at the given reference value was 42.74%, the specificity was 87.88%, and the ROC-AUC value was 0.766.

In another example, samples of urine from 124 cases of lung adenocarcinoma and 66 healthy subjects were subjected to an MRM method, thereby to determine the fragmentation rate of a protein fragment in which the sequence of C-terminal amino acids derived from kininogen I was IYPTVNCQPLG. When a patient's sample having a fragmentation rate that was 1.25 times or more higher than the mean value of the healthy subjects was considered to be positive for lung adenocarcinoma, 7 cases out of the healthy subjects and 78 cases out of the lung adenocarcinoma cases were positive for lung adenocarcinoma. When as a reference, the fragmentation rate was set to be 1.5 times the mean value of the healthy subjects group, 5 cases out of the healthy subjects cases and 68 cases out of the lung adenocarcinoma cases were positive for lung adenocarcinoma, and the sensitivity at the given reference value was the sensitivity at the given reference value was 54.84%, the specificity was 92.42%, and the ROC-AUC value was 0.814.

Samples of urine from 61 cases of early lung adenocarcinoma at stages IA and IB and 66 healthy subjects were subjected to an MRM method, thereby to determine the fragmentation rate of a protein fragment in which the sequence of C-terminal amino acids derived from kininogen I was ESNEELTESCET. When a patient's sample having a fragmentation rate that was 1.25 times or more higher than the mean value of the healthy subjects group was considered to be positive for lung adenocarcinoma, 8 cases out of the healthy subjects and 21 cases out of the early lung adenocarcinoma cases were positive for lung adenocarcinoma. When as a reference, the fragmentation rate was set to be 1.5 times the mean value of the healthy subjects group, 8 cases out of the healthy subjects and 18 cases out of the early lung adenocarcinoma cases were positive for lung adenocarcinoma, and the sensitivity at the given reference value was 29.51%, the specificity was 87.88%, and the ROC-AUC value was 0.676.

In another example, samples of urine from 61 cases of early lung adenocarcinoma at stages IA and IB and 66 healthy subjects were subjected to an MRM method, thereby to determine the fragmentation rate of a protein fragment in which the sequence of C-terminal amino acids derived from kininogen I was IYPTVNCQPLG. When a patient's sample having a fragmentation rate that was 1.25 times or more higher than the mean value of the healthy subjects group was considered to be positive for early lung adenocarcinoma, 7 cases out of the healthy subjects and 32 cases out of the early lung adenocarcinoma cases were positive for early lung adenocarcinoma. When as a reference, the fragmentation rate was set to be 1.5 times the mean value of the healthy subjects group, 5 cases out of the healthy subjects and 26 cases out of the early lung adenocarcinoma cases were positive for lung adenocarcinoma, and the sensitivity at the given reference value was the sensitivity at the given reference value was 42.62%, the specificity was 92.42%, and the ROC-AUC value was 0.766.

[Example 6] Detection by mass spectrometry

Samples of urine (~50 mL) were collected and markers derived from kininogen 1 were searched in a sequence of sample pretreatment, and obtaining and statistical analysis of analytical date. Sample pretreatment was performed to concentrate the urine samples 200 to 250 times using an Amicon Ultra-15 (having a molecular weight cut-off of 10 kDa) and an Amicon Ultra-4 (having a molecular weight cut-off of 10 kDa) (Merck Millipore), and remove low molecular weight molecules by washing three times with 3 mL of an aqueous solution of triethylammonium hydrogen carbonate containing 100 mM NaCl, prior to obtaining concentrated samples. The concentrated samples were subjected to protein quantification, and all the samples were adjusted to have a protein concentration, or a total protein amount, of 10 mg/mL using a buffer solution and used in a subsequent analysis step. Subsequently, the sample was subjected to reductive alkylation, followed by tryptic digestion in a buffer solution prepared using a particular concentration of $H_2^{18}O$ (a method for stable isotope labeling at the peptide C-terminus). After desalting and purification, trypsin-digested peptides were labeled by iTRAQ (8-plex, AB Sciex). Eight samples were mixed, and the mixture was desalted and purified. After that, only C-terminal trypsin-digested peptides derived from protein fragments were fractionated by ion-exchange chromatography (on an LC column of Poly-SULFOETHYL A™ (PolyLC Inc., USA) having an inner diameter of 4.6 mm and a length of 50 mm, at a flow rate of 0.4 mL/min, using as a solvent, a stepwise gradient of (from 0 to 100% of) an aqueous solution of 20% acetonitrile/5 mM monopotassium phosphate and 0.5M NaCl (pH2.55) in 20% acetonitrile/ phosphoric acid (pH2.55) for separating). The fraction of interest was desalted and purified, and then only the C-terminal trypsin-digested peptide was selected and extracted with Nano-LC (on an LC column having an ID of 75 um and a length of 100 mm and as a packing material, Inertsil C18 (a particle size of 3 um), at a flow rate of 250 nL/min, using as a solvent, a concentration gradient of (from 3 to 80% of) acetonitrile in 0.1% trifluoroacetic acid/water for separating) /MALDI-MS/MS(AB Sciex) (using an in-house developed program "iSpec"; see, Fernandez-de-Cossio J., Takao T. et al., Rapid Commun. Mass Spectrom. 18, 2465-2472 (2004)). The intensity of a control peak (with an m/z of 113, which was spiked in an equal amount in each assay) of the reporter peaks (with an m/z of 113-119, 121) involved in comparative quantification, which were contained in the MS/MS spectrum was used to normalize other reporter peaks before performing a comparison between assays. As a specific evaluation criterion, for qualitative analysis, MS/MS spectra in which the number of peaks having an intensity at or above a particular level was five or more were selected, and an assay was employed in which the number of proteins identified based on those MS/MS spectra was 50 or more. For quantitative analysis, use was made of a quantification value only from an assay in which the peak intensity of a reference reporter peak (with an m/z of 113) was greater than 10 and the number of MS/MS spectra was 300 or more.

[Example 7] Statistical analysis

Respective relative ratios to the peak intensity of a control sample were calculated for the peak intensity of each C-terminal trypsin-digested peptide in MS/MS spectra (Example 6) obtained from samples of urine from 58 cases of lung adenocarcinoma (5 cases at stage IA, 1 case at stage IB, 3 cases at stage IIIA, 8 cases at stage IIIb, and 41 cases at stage IV), 30 cases of benign respiratory diseases (bronchial asthma, empyema, lung fluke disease, nontuberculous mycobacteriosis, interstitial pneumonia, chronic obstructive pulmonary disease, sarcoidosis, inflammatory pseudotumor, bacterial pneumonia, allergic bronchopulmonary aspergillosis), and 25 cases of healthy subjects. A comparison was made between the groups of the lung adenocarcinoma cases and of the non-lung adenocarcinoma cases (benign respiratory diseases and healthy subjects). The peak detection rate of urine protein fragments derived from kininogen 1 in the groups of the 58 lung adenocarcinoma cases and of the 55 non-lung adenocarcinoma cases was tested by Fisher's exact test, and those giving a p value of less than 0.01 were extracted. Statistical analyses used JMP12 (SAS Institute Inc, Cary, NC). A protein fragment in which the sequence of C-terminal amino acids derived from kininogen 1 was TEHLASSSEDSTTPSA was detected in 26 cases out of the 58 lung adenocarcinoma cases and in 2 cases out of the 55 cases in the non-lung adenocarcinoma group, and met the above criterion (p value < 0.0001). When the peak intensity detected in the healthy subjects in the non-lung adenocarcinoma group was used as a reference, the sensitivity, the specificity, and the ROC-AUC value at the reference value were 37.93%, 96.36%, and 0.708.

[Example 8]

Respective relative ratios to the peak intensity of a control sample were calculated for the peak intensity of each C-terminal trypsin-digested peptide in MS/MS spectra (Example 6) obtained from samples of urine from 25 cases of early lung adenocarcinoma (18 cases at stage IA, 5 cases at stage IB, and 1 case at stage IIA) and 25 cases of healthy subjects. A comparison was made between the groups of the early lung adenocarcinoma cases and of the healthy subjects. For a protein fragment in which the sequence of C-terminal amino acids derived from kininogen 1 was IYPTVNCQPLGMIS, 10 cases out of the 24 cases of early lung adenocarcinoma gave a positive result when a reference value was used that was 1.25 times the peak intensity detected in the healthy subjects. The sensitivity at the given reference value was 41.7%, the specificity was 72%, and the ROC-AUC value was 0.644.

[Example 9]

Differently from Example 8, samples of urine from 37 cases of lung adenocarcinoma (15 cases at stage IA, 3 cases at stage IB, 4 cases at stage IIA, 2 cases at stage IIIA, 3 cases at stage IIIB, and 10 cases at stage IV) and 55 healthy subjects were subjected to an MRM method, thereby to determine the fragmentation rate of a protein fragment in which the sequence of C-terminal amino acids derived from kininogen I was IYPTVNCQPL. When a patient's sample having a fragmentation rate that was 1.25 times or more higher than the mean value of the healthy subjects group (0.005) was considered to be positive for lung adenocarcinoma, 12 cases out of the healthy subjects and 23 cases out of the lung adenocarcinoma cases were positive for lung adenocarcinoma. The sensitivity at the given reference value was the sensitivity at the given reference value was 63.2%, the specificity was 78.2%, and the ROC-AUC value was 0.757.

[Example 10]

In another example, samples of urine from 22 cases of early lung adenocarcinoma (15 cases at stage IA, 3 cases at stage IB, and 4 cases at stage IIA) and 55 healthy subjects were subjected to an MRM method, thereby to determine the fragmentation rate of a protein fragment in which the sequence of C-terminal amino acids derived from kininogen I was IYPTVNCQPL. When a patient's sample having a fragmentation rate that was 1.25 times or more higher than the mean value of the healthy subjects group (0.005) was considered to be positive for lung adenocarcinoma, 12 cases out of the healthy subjects and 16 cases out of the lung adenocarcinoma cases were positive for lung adenocarcinoma. The sensitivity at the given reference value was 72.7%, the specificity was 78.2%, and the ROC-AUC value was 0.842.

[Example 11]

In another example, samples of urine from 37 cases of lung adenocarcinoma (15 cases at stage IA, 3 cases at stage IB, 4 cases at stage IIA, 2 cases at stage IIIA, 3 cases at stage IIIB, and 10 cases at stage IV) and 55 healthy subjects were subjected to an MRM method, thereby to determine the fragmentation rate of a protein fragment in which the sequence of C-terminal amino acids derived from kininogen I was IYPTVNCQP. When a patient's sample having a fragmentation rate that was 1.25 times or more higher than the mean value of the healthy subjects group (0.044) was considered to be positive for lung adenocarcinoma, 11 cases out of the healthy subjects and 29 cases out of the lung adenocarcinoma cases were positive for lung adenocarcinoma. The sensitivity at the given reference value was the sensitivity at the given reference value was 73.7%, the specificity was 80%, and the ROC-AUC value was 0.865.

[Example 12]

In another example, samples of urine from 22 cases of early lung adenocarcinoma (15 cases at stage IA, 3 cases at stage IB, and 4 cases at stage IIA) and 55 healthy subjects were subjected to an MRM method, thereby to determine the fragmentation rate of a protein fragment in which the sequence of C-terminal amino acids derived from kininogen I was IYPTVNCQP. When a patient's sample having a fragmentation rate that was 1.25 times or more higher than the mean value of the healthy subjects group (0.044) was considered to be positive for lung adenocarcinoma, 11 cases out of the healthy subjects and 16 cases out of the lung adenocarcinoma cases were positive for lung adenocarcinoma. The sensitivity at the given reference value was 72.3%, the specificity was 80%, and the ROC-AUC value was 0.842.

[Example 13]

Samples of urine from 2 cases of sarcoma-like cancer (1 case of pleomorphic cancer and 1 case of carcinosarcoma) and 1 case of large cell carcinoma, who were histopathologically diagnosed via transbronchial biopsy or surgical lung biopsy, and 55 healthy subjects were each subjected to MRM, thereby to determine the fragmentation rate of a protein fragment in which the sequence of C-terminal amino acids derived from kininogen I was IYPTVNCQP. The fragmentation rate for each of the lung cancer cases was 0.125 for the pleomorphic cancer case, 0.162 for the carcinosarcoma case, and 0.135 for the large cell carcinoma case, and was higher than a reference value (0.055) that was 1.25 times the mean value of the healthy subjects group (0.044).

[Example 14]

Samples of urine from 4 cases of squamous cell carcinoma (2 cases at stage IB, 1 case at stage IIIA, and 1 case at stage IIIB) , who were histopathologically diagnosed via transbronchial biopsy or surgical lung biopsy, and 55 healthy subjects were each subjected to MRM, thereby to determine the fragmentation rate of a protein fragment in which the sequence of C-terminal amino acids derived from kininogen I was IYPTVNCQP. The fragmentation rates of a protein fragment in which the sequence of C-terminal amino acids from kininogen I was IYPTVNCQP, in the cases of squamous cell carcinoma were 0.098 and 0.090 for the stage IB cases, 0.19 for the stage IIIA case, and 0.44 for the stage IIIB case, and all the 4 cases of squamous cell carcinoma gave a higher fragmentation rate than a reference value (0.055) that was 1.25 times the mean value of the healthy subjects group (0.044).

[Example 15]

Samples of urine from 4 cases of small cell lung cancer, who were histopathologically diagnosed via transbronchial biopsy or surgical lung biopsy, and 55 healthy subjects were each subjected to MRM, thereby to determine the fragmentation rate of a protein fragment in which the sequence of C-terminal amino acids derived from kininogen I was IYPTVNCQP. The respective fragmentation rates of a protein fragment in which the sequence of C-terminal amino acids from kininogen I was IYPTVNCQP, in the cases of squamous cell carcinoma were 0.069, 0.131, 0.747, and 0.754, and all the 4 cases of small cell lung cancer gave a higher fragmentation rate than a reference value (0.055) that was 1.25 times the mean value of the healthy subjects group (0.044).

[Example 16] Detection by ELISA and comparison with serum CEA for usefulness

Samples of serum (10 $\mu$L) from 25 cases of early lung adenocarcinoma at stages IA and IB and 35 healthy subjects were subjected to an ELISA method, thereby to determine the concentration of a protein fragment in the serum, in which the sequence of C-terminal amino acids from kininogen I was IYPTVNCQPLG. To determine the concentration of the kininogen I-derived fragments in the serum, an antibody was used that specifically recognized a fragment derived from kininogen I in which the sequence of C-terminal amino acids was CQPLG, which was recovered from the serum of a chicken which had been immunized by administration of an prepared immunogen solution. When a subject's sample having a concentration of the protein fragment that was 1.2 times the mean of the healthy subjects was considered to be positive for early lung adenocarcinoma, 13 cases out of the 25 cases of early lung adenocarcinoma were allowed to be assessed to be positive for early lung adenocarcinoma. The sensitivity at the given reference value was 520, the specificity was 82.9%, and the ROC-AUC value was 0.706. When the reference value of serum CEA was set to be 5.0 ng/ml, on the other hand, only 7 cases out of the 25 cases of early lung adenocarcinoma gave a positive result. The sensitivity at the given reference value was 280, the specificity was 74.29%, and the ROC-AUC value was 0.534.

[Example 17] Changes in urine markers with the progression of lung adenocarcinoma

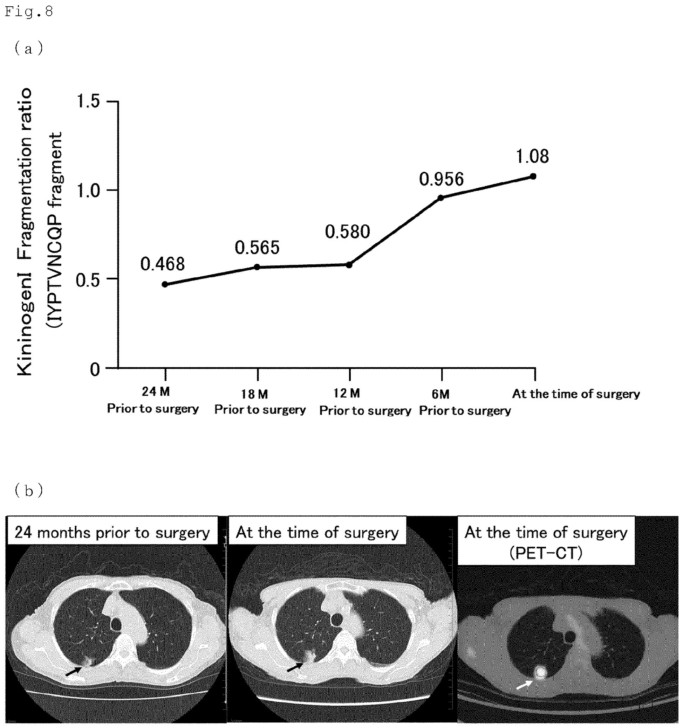

Samples of urine were collected over time from one case in which a 1.4-cm ground-glass nodular shadow suspected of lung adenocarcinoma was recognized in the periphery of the right lower lobe by a chest CT examination. The fragmentation rate of a protein fragment in which the sequence of C-terminal amino acids from kininogen I was IYPTVNCQP, and the size of the nodular shadow were compared with those at the time when the lung adenocarcinoma was diagnosed by surgical lung biopsy. The post-operative stage in this case was T1bN0M0, pStageIA. Results from the comparison of chest CT examinations between at 24 months prior to surgery and at the time thereof showed that the size of the nodular shadow was increased to 1.9 cm at the time of the surgery. In addition, the nodule was observed to have highly accumulated FDG in a PET-CT scan (Fig. 8). At 24, 18, 12, and 6 months prior to surgery, and at the time thereof, the fragmentation rate of the protein fragment in which the sequence of C-terminal amino acids from kininogen I was IYPTVNCQP was 0.468, 0.565, 0.58, 0.956, and 1.08, respectively, indicating that the fragmentation rate was increased with growing adenocarcinoma.

[Example 18] Changes in urine markers after lung adenocarcinoma resection

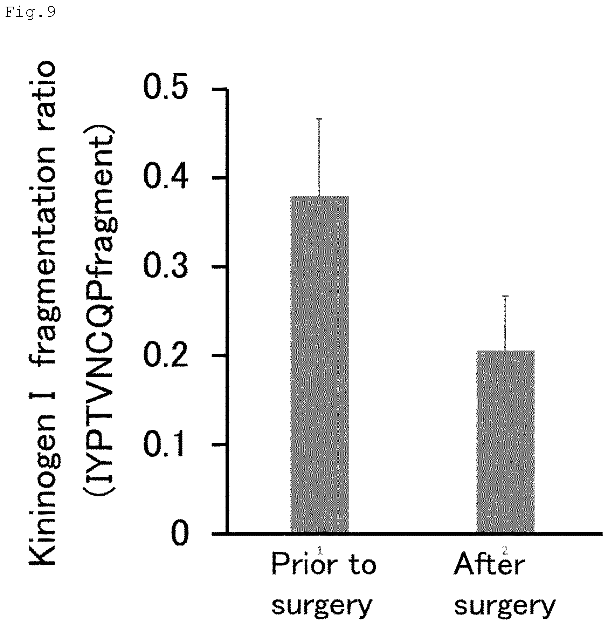

In 11 cases of early lung adenocarcinoma (5 cases at stage IA, 2 cases at stage IB, and 4 cases at stage IIA), samples of urine collected before and after lung cancer resection were subjected to an MRM method, thereby to determine the fragmentation rate of a protein fragment in which the sequence of C-terminal amino acids from kininogen I was IYPTVNCQP. For the analysis of urine samples after the surgery, samples of urine were used which had been collected at 14 days or later after the surgery. Samples of urine from 8 cases out of the 11 cases of early lung adenocarcinoma were found to exhibit a decrease in the fragmentation rate of the protein fragment in which the sequence of C-terminal amino acids from kininogen I was IYPTVNCQP. In the mean value of all the 11 cases, the fragmentation rate of the protein fragment in which the sequence of C-terminal amino acids from kininogen I was IYPTVNCQP was decreased from 0.378 to 0.205, by 45.77% (Fig. 9) .

[Example 19] Changes in urine markers by treatment of lung adenocarcinoma

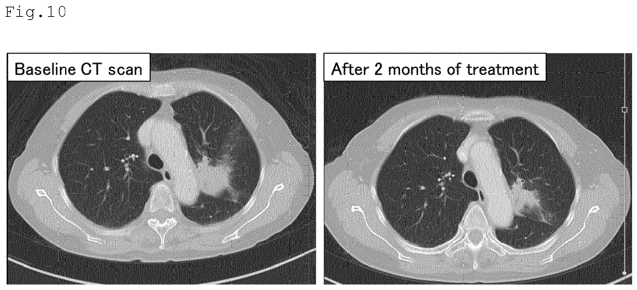

Samples of urine collected in 1 case of lung adenocarcinoma at stage IV who was positive for epidermal growth factor receptor (EGFR) gene mutation were subjected to an MRM method, whereby the fragmentation rate of a protein fragment in which the sequence of C-terminal amino acids from kininogen I was IYPTVNCQP was compared between before and at 2 months after a treatment with an EGFR tyrosine kinase inhibitor. The therapeutic effect by the EGFR tyrosine kinase inhibitor was observed to lead to a tumor reduction rate of 37.1% (Fig. 10), which met the criteria for Partial Response in the Response Evaluation Criteria In Solid Tumors (RECIST). The fragmentation rate of the protein fragment in which the sequence of C-terminal amino acids from kininogen I was IYPTVNCQP, at 2 months after the treatment, was decreased from 0.0783 to 0.0648, by 17.2%, compared with that before the treatment.

[Example 20]

In order to efficiently detect cleavage of kininogen I protein, two parameters were used: the amount of a trypsin-digested peptide having the amino acid sequence IYPTVNCQP or IYPTVNCQPL as a C-terminal portion (Cx), and the amount of a protein fragment having the amino acid sequence IYPTVNCQPLGMISLM as a C-terminal portion (Ix), to determine the fragmentation index (Fx) of kininogen I protein by the calculation formula described below. A patient's sample having a fragmentation index that was 1.25 times or more higher than the mean value of healthy subjects was considered to be positive for lung adenocarcinoma.

$$[Formula\ 2]\ Protein\ fragmentation\ index\ (Fx)\ =\ Cx/Ix$$

[Example 21]

Samples of urine from 27 cases of lung adenocarcinoma (13 cases at stage IA, 2 cases at stage IB, 4 cases at stage IIA, 2 cases at stage IIIA, and 6 cases at stage IV) and 49 healthy subjects were subjected to an MRM method, thereby to determine the fragmentation index (Fx) of a protein fragment in which the sequence of C-terminal amino acids from kininogen I was IYPTVNCQP. When a subject's sample having a fragmentation rate that was 1.25 times or more higher than the mean value of the healthy subjects group (4.82) was considered to be positive for lung adenocarcinoma, 14 cases out of the healthy subjects and 14 cases out of the lung adenocarcinoma cases were positive for lung adenocarcinoma, and the sensitivity at the given reference value was 71.4%, the specificity was 71.4%, and the ROC-AUC value was 0.829.

[Example 22]

Samples of urine from 19 cases of early lung adenocarcinoma (13 cases at stage IA, 2 cases at stage IB, and 4 cases at stage IIA) and 49 healthy subjects were subjected to an MRM method, thereby to determine the fragmentation index (Fx) of a protein fragment in which the sequence of C-terminal amino acids from kininogen I was IYPTVNCQP. When a subject's sample having a fragmentation rate that was 1.25 times or more higher than the mean value of the healthy subjects group (4.82) was considered to be positive for early lung adenocarcinoma, 14 cases out of the healthy subjects and 14 cases out of the lung adenocarcinoma cases were positive for early lung adenocarcinoma, and the sensitivity at the given reference value was 73.7%, the specificity was 71.4%, and the ROC-AUC value was 0.815.

[Example 23]

Samples of urine from 27 cases of lung adenocarcinoma (13 cases at stage IA, 2 cases at stage IB, 4 cases at stage IIA, 2 cases at stage IIIA, and 6 cases at stage IV) and 49 healthy subjects were subjected to an MRM method, thereby to determine the fragmentation index (Fx) of a protein fragment in which the sequence of C-terminal amino acids from kininogen I was IYPTVNCQPL. When a subject's sample having a fragmentation rate that was 1.25 times or more higher than the mean value of the healthy subjects group was considered to be positive for lung adenocarcinoma, 12 cases out of the healthy subjects and 21 cases out of the lung adenocarcinoma cases were positive for lung adenocarcinoma, and the sensitivity at the given reference value was 750, the specificity was 75.5%, and the ROC-AUC value was 0.848.

[Example 24]

Samples of urine from 19 cases of early lung adenocarcinoma (13 cases at stage IA, 2 cases at stage IB, and 4 cases at stage IIA) and 49 healthy subjects were subjected to an MRM method, thereby to determine the fragmentation index (Fx) of a protein fragment in which the sequence of C-terminal amino acids from kininogen I was IYPTVNCQPL. When a subject's sample having a fragmentation rate that was 1.25 times or more higher than the mean value of the healthy subjects group (0.437) was considered to be positive for early lung adenocarcinoma, 11 cases out of the healthy subjects and 13 cases out of the lung adenocarcinoma cases were positive for early lung adenocarcinoma, and the sensitivity at the given reference value was 68.4%, the specificity was 75.5%, and the ROC-AUC value was 0.860.

[Example 25]

Samples of urine from 15 cases of advanced lung adenocarcinoma (2 cases at stage IIIA, 3 cases at stage IIIB, and 10 cases at stage IV) and 55 healthy subjects were subjected to an MRM method, thereby to determine the amounts of presence of a protein fragment having the amino acid sequence IYPTVNCQPLGMISLM as a C-terminal portion from kininogen I and of a trypsin-digested peptide having the amino acid sequence YFIDFVAR as a C-terminal portion, to compare the ratio of presence of IYPTVNCQPLGMISLM, a C-terminal portion which is a normal protein fragment derived from kininogen I. When a patient's sample having a ratio of presence of the fragment that was 40% or more lower than the mean value of the healthy subjects group (0.0075) was considered to be positive for advanced lung adenocarcinoma, 15 cases out of the healthy subjects and 11 cases out of the advanced lung adenocarcinoma cases were positive for advanced lung adenocarcinoma. The sensitivity at the given reference value was 73.3%, and the specificity was 72.73%.

[Example 26]

Samples of urine from 34 cases of lung adenocarcinoma (14 cases at stage IA, 3 cases at stage IB, 3 cases at stage IIA, 2 cases at stage IIIA, 3 cases at stage IIIB, and 9 cases at stage IV) and 53 healthy subjects were subjected to an MRM method, thereby to calculate the ratio of peak intensity of a trypsin-digested peptide from amino acid positions 331 to 343 derived from kininogen I (ESNEELTESCETK) to that of a trypsin-digested peptide from amino acid positions 345 to 362 derived therefrom (LGQSLDCNAEVYVVPWEK) (which reflects the abundance of the former peptide to the latter). The samples from the lung cancer patients showed a smaller mean peak-intensity ratio of 0.150 relative to the mean value of the healthy subjects group (0.353). In the patient's samples, when the ratio of peak intensity of the trypsin-digested peptide LGQSLDCNAEVYWPWEK to that of the trypsin-digested peptide ESNEELTESCETK that was 40% or more lower than the mean of the healthy subjects was considered to be positive for lung adenocarcinoma, 6 cases out of the healthy subjects and 24 cases out of the lung adenocarcinoma cases were positive for lung adenocarcinoma. The sensitivity at the given reference value was 70.6%, the specificity was 88.68%, and the ROC-AUC value was 0.9.

[Example 27]

Samples of urine from 34 cases of lung adenocarcinoma (14 cases at stage IA, 3 cases at stage IB, 3 cases at stage IIA, 2 cases at stage IIIA, 3 cases at stage IIIB, and 9 cases at stage IV) and 53 healthy subjects were subjected to an MRM method, thereby to calculate the ratio of peak intensity of a trypsin-digested peptide from amino acid positions 209 to 219 derived from kininogen I (ENFLFLTPDCK) to that of a trypsin-digested peptide from amino acid positions 345 to 362 derived therefrom (LGQSLDCNAEVYVVPWEK) (which reflects the abundance of the former peptide to the latter). The samples from the lung cancer patients showed a smaller mean peak-intensity ratio of 2.0 relative to the mean value of the healthy subjects group (5.423). In the patient's samples, when the ratio of peak intensity of the trypsin-digested peptide ENFLFLTPDCK to that of the trypsin-digested peptide ESNEELTESCETK that was 40% or more lower than the mean of the healthy subjects was considered to be positive for lung adenocarcinoma, 3 cases out of the healthy subjects and 31 cases out of the lung adenocarcinoma cases were positive for lung adenocarcinoma. The sensitivity at the given reference value was 91.2%, the specificity was 94.34%, and the ROC-AUC value was 0.972.

**Claims**

1. A method for detection of lung cancer, comprising a step of detecting in vitro the presence or absence of abnormal cleavage of kininogen I in a subject-derived sample; wherein the step of detecting in vitro the presence or absence of abnormal cleavage of kininogen I comprises detecting the amount or presence of at least any one selected from the group consisting of:

(a) a protein fragment having ESNEELTESCET as a C-terminal amino acid sequence;
(b) a protein fragment having IYPTVNCQPLG as a C-terminal amino acid sequence;
(c) a protein fragment having TEHLASSSEDSTTPSA as a C-terminal amino acid sequence;
(d) a protein fragment having IYPTVNCQPLGMIS as a C-terminal amino acid sequence;
(e) a protein fragment having IYPTVNCQPL as a C-terminal amino acid sequence; and
(f) a protein fragment having IYPTVNCQP as a C-terminal amino acid sequence.

2. The method for detection of lung cancer according to claim 1, wherein the subject-derived sample is selected from urine or blood.

3. The method for detection of lung cancer according to any one of claims 1 and 2, comprising a step of using at least one method selected from the group consisting of a mass spectrometric method, an immunochemical method, and a chromatographic method.

4. The method for detection of lung cancer according to any one of claims 1 to 3, wherein the lung cancer is a non-small cell cancer selected from the group consisting of adenocarcinoma, squamous cell carcinoma, large cell carcinoma, adenosquamous carcinoma, pleomorphic cancer, and carcinosarcoma, or is small cell lung cancer.

5. The method for detection of lung cancer according to claim 1, wherein the presence or absence of abnormal cleavage in the kininogen I is determined by a fragmentation rate of the kininogen I, the fragmentation rate being given by:

kininogen I protein fragmentation rate $(F_n) = C_n/I_n$
where
"$C_n$" is the amount of each protein fragment from kininogen I, and
"$I_n$" is the total amount of proteins derived from the kininogen I protein.

6. The method for detection of lung cancer according to any one of claims 1 to 5, wherein the method is directed to any one or more selected from the group consisting of a detection for assisting the diagnosis of early lung cancer, a detection for the diagnosis of lung cancer, a detection of advanced lung cancer, a detection for predicting the presence or absence of recurred lung cancer, and a detection on the presence or absence of therapeutic effects against lung cancer.

**Patentansprüche**

1. Verfahren zum Nachweis von Lungenkrebs, umfassend einen Schritt eines Nachweisens des Vorliegens oder des Fehlens einer anomalen Spaltung von Kininogen I in einer von einem Patienten stammenden Probe in vitro; wobei der Schritt des Nachweisens des Vorliegens oder des Fehlens einer anomalen Spaltung von Kininogen I in vitro ein Nachweisen der Menge oder des Vorliegens von mindestens einem beliebigen umfasst, das ausgewählt ist aus der Gruppe bestehend aus:

(a) einem Proteinfragment mit ESNEELTESCET als eine C-terminale Aminosäuresequenz;
(b) einem Proteinfragment mit IYPTVNCQPLG als eine C-terminale Aminosäuresequenz;
(c) einem Proteinfragment mit TEHLASSSEDSTTPSA als eine C-terminale Aminosäuresequenz;
(d) einem Proteinfragment mit IYPTVNCQPLGMIS als eine C-terminale Aminosäuresequenz;
(e) einem Proteinfragment mit IYPTVNCQPL als eine C-terminale Aminosäuresequenz;
(f) einem Proteinfragment mit IYPTVNCQP als eine C-terminale Aminosäuresequenz.

2. Verfahren zum Nachweis von Lungenkrebs nach Anspruch 1, wobei die von einem Patienten stammende Probe aus Urin oder Blut ausgewählt ist.

3. Verfahren zum Nachweis von Lungenkrebs nach einem der Ansprüche 1 und 2, umfassend einen Schritt eines Verwendens von mindestens einem Verfahren, das ausgewählt ist aus der Gruppe bestehend aus einem massenspektrometrischen Verfahren, einem immunochemischen Verfahren und einem chromatographischen Verfahren.

4. Verfahren zum Nachweis von Lungenkrebs nach einem der Ansprüche 1 bis 3, wobei der Lungenkrebs ein nicht-kleinzelliger Krebs ist, der ausgewählt ist aus der Gruppe bestehend aus Adenokarzinom, Plattenepithelkarzinom, großzelligem Karzinom, adenosquamösem Karzinom, pleomorphem Krebs und Karzinosarkom, oder kleinzelliger Lungenkrebs ist.

5. Verfahren zum Nachweis von Lungenkrebs nach Anspruch 1, wobei das Vorliegen oder das Fehlen einer anomalen Spaltung in dem Kininogen I durch eine Fragmentationsrate des Kininogens I bestimmt wird, wobei die Fragmentationsrate gegeben ist durch:

Kininogen-I-Protein-Fragmentationsrate ($F_n$) = $C_n/I_n$,
wobei
"$C_n$" die Menge jedes Proteinfragments von Kininogen I ist und
"$I_n$" die Gesamtmenge von Proteinen ist, die von dem Kininogen-I-Protein stammen.

6. Verfahren zum Nachweis von Lungenkrebs nach einem der Ansprüche 1 bis 5, wobei das Verfahren auf einen beliebigen oder beliebige mehrere abgezielt ist, die ausgewählt sind aus der Gruppe bestehend aus einem Nachweis zum Unterstützen der Diagnose von frühem Lungenkrebs, einem Nachweis zur Diagnose von Lungenkrebs, einem Nachweis von fortgeschrittenem Lungenkrebs, einem Nachweis zur Vorhersage des Vorliegens oder des Fehlens von wiedergekehrtem Lungenkrebs und einem Nachweis des Vorliegens oder des Fehlens von therapeutischen Wirkungen auf Lungenkrebs.

**Revendications**

1. Procédé pour la détection d'un cancer du poumon, comprenant une étape de détection in vitro de la présence ou de l'absence d'un clivage anormal du kininogène I dans un échantillon dérivé d'un sujet ; dans lequel l'étape de détection in vitro de la présence ou de l'absence d'un clivage anormal du kininogène I comprend la détection de la quantité ou de la présence d'au moins l'un quelconque choisi dans le groupe constitué par :

(a) un fragment protéique ayant ESNEELTESCET en tant que séquence d'acides aminés C-terminale ;
(b) un fragment protéique ayant IYPTVNCQPLG en tant que séquence d'acides aminés C-terminale ;
(c) un fragment protéique ayant TEHLASSSEDSTTPSA en tant que séquence d'acides aminés C-terminale ;
(d) un fragment protéique ayant IYPTVNCQPLGMIS en tant que séquence d'acides aminés C-terminale ;
(e) un fragment protéique ayant IYPTVNCQPL en tant que séquence d'acides aminés C-terminale ; et
(f) un fragment protéique ayant IYPTVNCQP en tant que séquence d'acides aminés C-terminale.

2. Le procédé pour la détection d'un cancer du poumon selon la revendication 1, dans lequel l'échantillon dérivé d'un sujet est choisi parmi l'urine et le sang.

3. Le procédé pour la détection d'un cancer du poumon selon l'une quelconque des revendications 1 et 2, comprenant une étape d'utilisation d'au moins un procédé choisi dans le groupe constitué par un procédé spectrométrique de masse, un procédé immunochimique, et un procédé chromatographique.

4. Le procédé pour la détection d'un cancer du poumon selon l'une quelconque des revendications 1 à 3, dans lequel le cancer du poumon est un cancer non à petites cellules choisi dans le groupe constitué par un adénocarcinome, un carcinome à cellules squameuses, un carcinome à grosses cellules, un carcinome adénosquameux, un cancer pléomorphe, et un carcinosarcome, ou est un cancer du poumon à petites cellules.

5. Le procédé pour la détection d'un cancer du poumon selon la revendication 1, dans lequel la présence ou l'absence d'un clivage anormal dans le kininogène I est déterminé par le taux de fragmentation du kininogène, le taux de fragmentation étant donné par :

taux de fragmentation de protéine kininogène I ($F_n$) = $C_n/I_n$ où
"$C_n$" est la quantité de chaque fragment protéique provenant du kininogène I, et
"$I_n$" est la quantité totale de protéines dérivant de la protéine kininogène I.

6. Le procédé pour la détection d'un cancer du poumon selon l'une quelconque des revendications 1 à 5, lequel procédé concerne un ou plusieurs choisis dans le groupe constitué par une détection pour faciliter le diagnostic d'un cancer du poumon précoce, une détection pour le diagnostic d'un cancer du poumon, une détection d'un cancer du poumon avancé, une détection pour prédire la présence ou l'absence d'une rechute de cancer du poumon, et une détection sur la présence ou l'absence d'effets thérapeutiques contre un cancer du poumon.

Fig.1

Fig.2

Fig.3

Fig.4

Fig.5

Fig.6

Fig.7

Fig.8

(a)

(b)

Fig.9

Fig.10

Baseline CT scan | After 2 months of treatment

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012526976 W
- JP 2014115186 A

### Non-patent literature cited in the description

- **SAWABATA N et al.** *J Thorac Oncol.,* 2011, vol. 6, 1229-35
- **ATAGI S et al.** *Lancet Oncol.,* 2012, vol. 13, 671-8
- **SCAGLIOTTI GV et al.** *J Clin Oncol.,* 2008, vol. 26, 3543-51
- **MOLINA R et al.** *Am J Respir Crit Care Med.,* 2016, vol. 193, 427-37
- **MATSUOKA K et al.** *Eur J Cardiothorac Surg.,* 2007, vol. 32, 435-9
- **RASTEL D et al.** *Eur J Cancer.,* 1994, vol. 30A, 601-6
- **STIEBER P et al.** *Anticancer Res.,* 1999, vol. 19 (4A), 2673-8
- **DI MATTEI et al.** Biochemical Pharmacology. Elsevier, 01 May 1967, vol. 16, 909-911
- **TRIFILIEFF A et al.** British Journal of Pharmacology. Wiley-Blackwell, 01 January 1994, vol. 111, 1228-1232
- **DA COSTA PATRICIA L N et al.** *Cancer Letter, New York, NY, US,* 11 December 2013, vol. 345 (1), 27-38
- **D. CHAN et al.** *Proceedings of the National Academy of Sciences,* 02 April 2003, vol. 99 (7), 4608-4613
- **CLAUDIA LANDI et al.** Journal of Clinical Bioinformatics. Biomed Central LTD, 10 November 2011, vol. 1, 31
- General Rule for Clinical and Pathological Record of Lung Cancer. Kanehara & Co., Ltd,
- **FERNANDEZ-DE-COSSIO J. ; TAKAO T. et al.** *Rapid Commun. Mass Spectrom.,* 2004, vol. 18, 2465-2472